# EUROPEAN PATENT SPECIFICATION

(11) **EP 0 461 850 B1**
(45) Date of publication and mention of the grant of the patent: **27.09.1995**
(21) Application number: 91305240.3
(22) Date of filing: 11.06.1991
(51) Int. Cl.: C07C 231/06

(54) **Process for producing alpha-hydroxycarboxylicacid amides**
Verfahren zur Herstellung von alpha-Hydroxycarbonsäure amiden
Procédé de préparation d'amides d'acides alpha-hydroxycarboliques

(30) Priority: 11.06.1990 JP 149960/90
(43) Date of publication of application: 18.12.1991
(73) Proprietor: MITSUI TOATSU CHEMICALS, Inc., Chiyoda-Ku Tokyo 100 (JP)
(72) Inventor: Karasawa, Minato, Mobara-shi, Chiba-ken (JP); Kageyama, Hiroharu, Mobara-shi, Chiba-ken (JP); Tokunoh, Sinji, Mobara-shi, Chiba-ken (JP); Inomata, Masamitsui, Mobara-shi, Chiba-ken (JP); Tokumitsu, Masahiro, Mobara-shi, Chiba-ken (JP); Miyama, Kanemitsu, Mobara-shi, Chiba-ken (JP)
(74) Representative: Nicholls, Kathryn Margaret

(56) References cited:
- EP-A- 0 418 512
- FR-A- 2 103 663
- US-A- 3 699 164
- US-A- 3 794 682
- DERWENT WPIL ONLINE ABSTRACT, accession no. 88-108793, Derwent Publications Ltd, London, GB; & JP-A-63 057 535 (MITSUI TOATSU CHEM. INC.)
- DERWENT WPIL ONLINE ABSTRACT, accession no. 88-108792, Derwent Publications Ltd, London, GB; & JP-A-63 057 534 (MITSUI TOATSU CHEM. INC.)

## Description

The present invention relates to a process for producing amide compounds by reacting nitrile compounds with water in a liquid phase.

Amide compounds are very useful as intermediates for organic syntheses which can be starting materials for agricultural chemicals, pharmaceuticals, synthetic resins and the like. In particular, methyl methacrylate can be produced by a vapor phase reaction of α-hydroxyisobutyramide (hereinafter referred to as "HAM") in the presence of methyl alcohol and a solid acid catalyst (Japanese Patent Application Pubication Nos. 10,940/1988 and 63,537/1988).

It is known that amide compounds can be produced by the reaction of the corresponding nitrile compounds with water and further, various catalysts useful for this reaction are also known. U.S. Pat. No. 3,366,639 discloses a manganese oxide which is one of such catalysts. Copper-containing catalysts often used for the hydration reaction of nitrile compounds can give only an insufficient effect in hydration of α-hydroxynitrile compounds such as acetone cyanohydrin (hereinafter referred to as "ACH") and the like. On the contrary, manganese oxides give fairly good results in hydration of α-hydroxynitrile compounds as shown in West German Patent No. 2,131,813. However, as is clear from the description in Japanese Patent Application Laid-open No. 222/1977, a particular skill is necessary for preparing the manganese oxides active in hydration of nitrile compounds disclosed in West German Patent No. 2,131,813, and moreover, there is a problem that the characteristics of the catalyst produced are different from batch to batch.

EP-A-418,512 was published after the priority date of the present application and is therefore relevant only to the novelty of the present invention, under Article 54(3) EPC. It describes a process for producing an α-hydroxycarboxylic acid amide by hydrating a cyanhydrin in the presence of a modified manganese oxide catalyst. The catalyst contains an alkali metal element, and one or more of zirconium, vanadium and tin.

US-A-3,794,682 discloses catalysts for use in the hydration of nitriles to amides, the catalysts comprising cobalt oxide and mixed metal oxides of cobalt and another metal selected from iron, aluminium, zinc, calcium, manganese and mercury. The mixed metals are preferably coprecipitated as oxalates, then decomposed by heating, to make the catalysts.

US-A-3,699,164 also describes a catalytic process for hydration of a nitrile, using a manganese dioxide catalyst. The process is carried out in a dilute aqueous solution of a strong acid, at a pH below 3.0, in order to improve conversion and increase the catalyst life.

In some embodiments the present invention may provide a process for producing α-hydroxycarboxylic acid amides by hydration of α-hydroxynitriles efficiently in the presence of a manganese dioxide catalyst.

In some embodiments the present invention may also provide a manganese dioxide catalyst substantially free from fluctuation in catalytic characteristics in the hydration of α-hydroxynitriles.

Embodiments of the invention may provide a manganese dioxide catalyst having an improved catalytic activity in the hydration of α-hydroxynitriles.

Further, embodiments of the present invention may provide a manganese dioxide catalyst having a long catalyst life which is important as an industrial catalyst.

According to the present invention, there is provided a process for producing an α-hydroxycarboxylic acid amide which comprises reacting an α-hydroxynitrile compound with water in a liquid phase in the presence of a catalyst consisting essentially of manganese dioxide containing an element selected from the group consisting of Groups IIIA, VA (excluding nitrogen), IIIB, VB, VIB and VIII of the Periodic Table, carbon, silicon, germanium, lead and mixtures thereof, the manganese dioxide having been prepared by reacting permanganate with manganese sulphate under acidic conditions, the element contained in the manganese dioxide being present in an amount of from 0.005 to 0.5 atoms per atom of manganese and having been incorporated into the manganese dioxide by impregnation or kneading.

### DESCRIPTION OF THE PREFERRED EMBODIMENTS

The nitrile compound used in the present invention is an α-hydroxynitrile compound represented by the following formula:

R · CN

where R is selected from alkyl, cycloalkyl, alkenyl, cycloalkenyl, aryl, alkaryl, aralkyl and heterocyclic groups substituted at the α-position with a hydroxy group. R may otherwise be unsubstituted, or substituted with halogen, alkoxy, nitro, ester, ketone or hydroxy group(s). The nitrile may in particular be a ketone cyanohydrin such as acetone cyanohydrin (ACH). Further, polynitriles may also be used.

The manganese dioxide used in the present invention may be anhydrous or hydrated. The manganese dioxide is produced by for instance reacting potassium or sodium permanganate and manganese sulfate in an acidic condition (J. Chem. Soc., 1953, p. 2189 (1953)).

A manganese oxide catalyst prepared by reducing a permanganate with a hydrohalic acid is disclosed in Japanese Patent Application Laid-open No. 57,535/1988, for use in converting e.g. acetone cyanohydrin into alpha-hydroxyisobutylamide in aqueous conditions.

Examples of elements of Groups IIIA, VA, IIIB, VB, VIB and VIII include boron, aluminium, gallium, indium, thallium; phosphorus, arsenic, antimony, bismuth; scandium, yttrium, lanthanum, cerium, praseodymium, neodymium, samarium, thorium; vanadium, niobium, tantalum; chromium, molybdenum, tungsten; and iron, ruthenium, osmium, cobalt, rhodium, iridium, nickel, palladium, platinum. Carbon, silicon, germanium and lead can also be used in the catalysts of the present invention.

One or more of these elements are incorporated in manganese dioxide.

Among them, manganese dioxide containing at least one element of Groups IIIA, VA, IIIB, and VIII of the Periodic Table is preferable.

Aluminium is preferable in Group IIIA; lead in the group consisting of carbon, silicon, germanium, lead and mixtures thereof; antimony and bismuth in Group VA; scandium and lanthanum in Group IIIB; and iron, cobalt and nickel in Group VIII.

The elements to be incorporated in the manganese dioxide catalyst are usually used in the form of nitrate, chloride, sulfate, carbonate, phosphate, hydroxide, oxide, simple substance or the like.

The amount of the element contained in manganese dioxide is 0.005 - 0.50 atom, preferably 0.01 - 0.2 atom, per atom of the total manganese.

Salts, hydroxides, oxides, simple substance or the like of elements of Groups IIIA, VA, IIIB, VB, VIB and VIII of the Periodic Table, and/or carbon, silicon, germanium and lead, may be incorporated in the manganese dioxide by impregnation or kneading. In particular, it is preferred to impregnate manganese dioxide with a salt of the element and neutralize with an alkali or the like.

The catalyst solution is filtered and the filter cake is washed with water and then dried at 80 - 200°C in air. The resulting catalyst is shaped into the desired form suitable for a hydration reaction in which said catalyst is to be used. It is particularly preferable to incorporate the element in the form of hydroxide and/or oxide in manganese dioxide according to the above-mentioned preparation method.

The amount of the catalyst used in the present invention is usually 0.01 - 0.50 part by weight per one part of the starting material, i.e. the nitrile compound, in the case of batch system. The concentration of the catalyst in a reaction vessel is usually 1 % by weight or more, preferably 2 - 30 % by weight in the case of a continuous reaction system of a fluidized catalyst bed type.

The amount of water used for the hydration reaction of the nitrile compound in the present invention is usually one mole or more, preferably 5 - 30 moles per one mole of the nitrile group of the nitrile compound.

As a reaction solvent, water is usually used, but when the nitrile compound is hydrophobic, a solvent capable of enhancing the compatibility with water is used which may be, for example, lower alcohols such as methyl alcohol, ethyl alcohol and the like, ethers such as 1,4-dioxane, tetrahydrofuran and the like, ketones such as acetone and the like, dimethyl-sulfoxide, N,N-dimethylformamide, N,N-dimethylacetamide, N-methyl-pyrrolidone and the like.

In particular, when a ketone cyanohydrin is used as the nitrile compound, it is desirable to use usually 0.1 - 3.0 moles of the ketone constituting the nitrile compound per one mole of the nitrile compound in addition to the above-mentioned reaction solvent in order to suppress the decomposition of the ketone cyanohydrin. For example, when an α-hydroxynitrile compound such as ACH is used, it is desirable to add acetone as shown in Japanese Patent Application Laid-open No. 222/1977.

The reaction temperature is usually 0 - 200°C, preferably 10 - 150°C, more preferably 30 - 80°C.

The reaction pressure is not critical as long as the pressure is sufficient to keep the reactants in a liquid state at the reaction temperature. That is, reduced pressure, atmospheric pressure or high pressure may be used provided that the above conditions are satisfied.

The process of the present invention is carried out in a liquid phase and may be effected batchwise or continuously. In the case of a continuous system, there may be used a fluidized catalyst bed or a fixed catalyst bed. When a reactor of a fluidized catalyst bed type is used, the reactor outlet is provided with a metal or glass filter or the like so that the ground catalyst particles may not flow out.

The liquid raw materials, i.e. nitrile compound, water and reaction solvent, may be fed to the reactor in the form of a mixture thereof or separately.

The residence time of the reaction fluid in the reactor may be appropriately set so as to convert an α-hydroxynitrile compound to the corresponding amide compound in high conversion and high selectivity.

The amide product solution obtained in high yield flowing out from the reactor is subjected to distillation to distill away the solvent and the resulting product is crystallized and separated in water, ether and/or acetone solvents to recover the end product, the amide compound.

According to the present invention, hydration of α-hydroxynitriles can be efficiently carried out in the presence of a manganese dioxide catalyst to produce α-hydroxycarboxylic acid amides.

The manganese dioxide catalyst containing the specified element exhibits catalytic characteristics which are substantially free from fluctuation, and has high catalytic activity and/or a very long catalyst life which is an important factor when used in an industrial process.

The present invention will be explained further in detail referring to the following examples and comparison examples.
The "%" in the following is molar percent unless otherwise specified.

### (1) Preparation of manganese dioxide catalysts containing the second element and activity test of the catalysts

1) Comparison Example - Preparation of manganese dioxide using coprecipitation method: To one liter of an aqueous solution of manganese (II) sulfate (395 g/liter) was added sulfuric acid to prepare an aqueous solution of manganese (II) sulfate of pH 1. To the resulting aqueous solution was added 278.6 g of potassium permanganate to oxidize the manganese (II) sulfate and one liter of water was added to the resulting slurry and aged for 30 min. while keeping the temperature at about 50°C. The product thus aged was subjected to vacuum filtration by an aspirator and then washed with one liter of 7 % (by weight) aqueous ammonia and 3 liters of water to obtain manganese dioxide.
2) Preparation of manganese dioxide catalysts containing the second element (Impregnation or kneading)
   Catalyst A₁:
   2.45 g of ferric nitrate nonahydrate was dissolved in 100 g of distilled water and to the resulting aqueous solution, 10 g of manganese dioxide (atomic ratio of Fe/Mn = 0.053) prepared as above was added and the pH of the resulting solution was adjusted to 7 with 28 % (by weight) aqueous ammonia. Then the aqueous solution was concentrated to dryness.
   The resulting solid matter was dried at 120°C for 5 hours, and then calcined at 400°C for 4 hours to obtain Catalyst A₁.
   Catalyst B:
   Using cobalt nitrate hexahydrate 1.67 g (atomic ratio of Co/Mn = 0.050),
   prepared in the same manner as Catalyst A₁.
   Catalyst C:
   Using nickel nitrate hexahydrate 3.35 g (atomic ratio of Ni/Mn = 0.100),
   prepared in the same manner as Catalyst A₁.
   Comparison Catalyst D:
   Using sodium nitrate 0.05 g (atomic ratio of Na/Mn = 0.005),
   prepared in the same manner as Catalyst A₁.
   Comparison Catalyst E:
   Using anhydrous barium chloride 0.48 g (atomic ratio of Ba/Mn = 0.020),
   prepared in the same manner as Catalyst A₁.
   Catalyst F:
   Using anhydrous aluminum chloride 3.07 g (atomic ratio of Al/Mn = 0.200),
   prepared in the same manner as Catalyst A₁.
   Comparison Catalyst G:
   Using tin tetrachloride 1.50 g (atomic ratio of Sn/Mn = 0.050),
   prepared in the same manner as Catalyst A₁.
   Catalyst H:
   Prepared by sufficiently kneading 0.41g of graphite with 10g of manganese dioxide prepared as above (atomic ratio of C/Mn = 0.300) followed by drying at 120°C for 9 hours.
   Catalyst I:
   Using scandium nitrate tetrahydrate 0.35 g (atomic ratio of Sc/Mn = 0.010),
   prepared in the same manner as Catalyst A₁.
   Catalyst J:
   Using lanthanum nitrate hexahydrate 2.62 g (atomic ratio of La/Mn = 0.053),
   prepared in the same manner as Catalyst A₁.
   Comparison Catalyst K:
   Using titanium tetrachloride 2.19 g (atomic ratio of Ti/Mn = 0.100), prepared in the same manner as Catalyst A₁.
   Comparison Catalyst L:
   Using zirconiun oxychloride octahydrate 1.95 g (atomic ratio of Zr/Mn = 0.053),
   Prepared in the same manner as Catalyst A₁.
   Catalyst M:
   Using vanadium oxytrichloride 1.00 g (atomic ratio of V/Mn = 0.050),
   prepared in the same manner as Catalyst A₁.
   Catalyst N:
   Using chromium nitrate nonahydrate 4.60 g (atomic ratio of Cr/Mn = 0.100),
   prepared in the same manner as Catalyst A₁.
   Catalyst P:
   Using bismuth nitrate pentahydrate 5.58 g (atomic ratio of Bi/Mn = 0.100),
   prepared in the same manner as Catalyst A₁.
   Catalyst A₂:
   The procedure for the preparation of Catalyst A₁ was repeated except that ferric nitrate nonahydrate was not added, Catalyst A₂ was obtained.
3) Activity test of catalyst
   Comparison Example 1
   In a 100 ml round bottom flask equipped with a reflux condenser and a thermometer were placed the above-mentioned Catalyst A₂ 4.8 g, ACH 16.0 g, acetone 16.5 g, and water 16.9 g, and a reaction was carried out with stirring at 40°C for 3 hours. In the liquid raw material mixture, the molar ratio of ACH: acetone: water was 1 : 1.5 : 5 and the pH was 2.8.
   The catalyst was filtered off from the reaction fluid and then the catalyst was washed with water. A mixture of the filtrate and the rinsing water was analyzed by gas chromatography. The conversion of ACH was 33.4 % and the selectivity of HAM was 85.7 %.
   Comparison Example 2
   The procedure of Comparison Example 1 was repeated by using the same catalyst A₂. The result is shown in Table 1. As is clear from Table 1, there is fluctuation in the catalyst characteristics between Comparison Examples 1 and 2 where the same Catalyst A₂ was used and the catalytic activity is poor.
   Examples 1 - 15
   The procedure of Comparison Example 1 was repeated except that Catalyst A₂ was replaced with one of Catalyst A₁ and Catalyst B to Catalyst P as mentioned above.

The results shown in Table 1 below indicate that the catalytic characteristics of the manganese dioxide catalyst containing an element selected from Groups IIIA, VA (other than nitrogen), IIIB, VB, VIB, and VIII of the Periodic Table, and/or carbon, silicon, germanium and lead, are markedly improved as compared with those of the manganese dioxide catalyst devoid of such element.

Therefore, it will be noted that the present invention is very useful for hydration of α-hydroxynitrile compounds such as ACH and the like.

### (2) Comparative data - Life test of manganese dioxide catalyst containing the second element (prepared by coprecipitation method)

1) Preparation of catalyst:
   To one liter of an aqueous solution of manganese (II)sulfate (395 g/liter) was added sulfuric acid to prepare an aqueous solution of manganese (II) sulfate of pH 1.
   To the resulting solution was added a salt of the respective element such that the respective element was present in an amount of 0.02 - 0.20 atom per one atom of the total manganese (Mn(II)+Mn(VII)). To the resulting solution was added 278.6 g of potassium permanganate to oxidize the manganese compound, and then one liter of water was added to the resulting slurry, which was then aged for 30 min. while keeping the temperature at about 50°C. To the solution thus aged was added 28 % (by weight) aqueous ammonia to adjust the pH of the solution to 7 followed by stirring for one hour. The resulting solution was vacuum filtered using an aspirator, and the filter cake was washed with 3 liter of water and dried at 120°C for 12 hours to obtain a manganese dioxide catalyst containing the second element.
   Further, the above-mentioned procedure was repeated except that the salt of the second element was not added, and a manganese dioxide catalyst void of the second element was prepared.
2) Life test of catalyst in the continuous hydration reaction of ACH
   Comparison Examples 3 - 5 and Examples 16 - 28
   A pressure resistant reactor (inner volume 50 ml; equipped with a glass stirrer, a mercury thermometer, a raw material feeding opening, and a liquid outlet provided with a glass-ball-filter) was charged with 30 g of manganese dioxide catalyst of 60 - 100 mesh and 300 g of water and the inner temperature was kept at 60°C.
   Then, a liquid raw material mixture (molar ratio of ACH: acetone: water being 1 : 1.5 : 18) was fed to the reactor at a flow rate of 37 ml/hr by a quantitative pump and the continuous hydration reaction was carried out for 20 days. The amount of liquid in the reactor was in the range of 290 - 310 ml.
   As manganese dioxide catalysts, the manganese dioxide catalysts containing the second element and the manganese dioxide catalyst void of the second element prepared as mentioned above were used respectively.
   The results are shown in Table 2.

**Table 1**

| Test of Activity of Manganese Dioxide Catalyst containing Second Element | | | | |
|---|---|---|---|---|
| | Added Element | Atomic Ratio of Added Element to Manganese | ACH Conversion (%) | HAM Selectivity (%) |
| Comparison Example 1 | None | 0 | 33.4 | 85.7 |
| Comparison Example 2 | None | 0 | 18.2 | 80.7 |
| Example 1 | Fe | 0.053 | 93.1 | 96.8 |
| Example 2 | Co | 0.050 | 92.6 | 96.8 |
| Example 3 | Ni | 0.100 | 91.4 | 95.7 |
| Comparison Example 4 | Na | 0.005 | 89.8 | 94.9 |
| Comparison Example 5 | Ba | 0.020 | 92.5 | 96.1 |
| Example 6 | Al | 0.200 | 94.1 | 96.0 |
| Comparison Example 7 | Sn | 0.050 | 90.7 | 95.3 |
| Example 8 | C | 0.300 | 93.3 | 95.8 |
| Example 9 | Sc | 0.010 | 87.3 | 95.3 |
| Example 10 | La | 0.053 | 87.2 | 94.6 |
| Comparison Example 11 | Ti | 0.100 | 92.0 | 95.7 |
| Comparison Example 12 | Zr | 0.053 | 90.9 | 95.2 |
| Example 13 | V | 0.050 | 87.5 | 94.0 |
| Example 14 | Cr | 0.100 | 87.0 | 93.8 |
| Example 15 | Bi | 0.100 | 95.6 | 99.0 |

**Table 2**

| Life Test of Manganese Dioxide containing Second Element (Comparative data) | | | | | | |
|---|---|---|---|---|---|---|
| | Salt of Added Element | Atomic Ratio of Added Element to Manganese | HAM Yield (%) | | | |
| | | | After 1 day | After 7 days | After 12 days | After 20 days |
| Comparison Example 3 | None | 0 | 84 | 83 | 29 | 7 |
| Example 16 | Fe(NO₃)₂ | 0.10 | 93 | 92 | 90 | 92 |
| Example 17 | Co(NO₃)₂ | 0.10 | 92 | 92 | 91 | 92 |
| Example 18 | Ni(NO₃)₂ | 0.10 | 91 | 90 | 90 | 90 |
| Comparison Example 4 | Na₂SO₄ | 0.10 | 89 | 89 | 60 | 19 |
| Comparison Example 5 | Ca(NO₃)₂ | 0.10 | 90 | 90 | 48 | 22 |
| Example 19 | Al(NO₃)₃ | 0.20 | 93 | 92 | 75 | 61 |
| Example 20 | SnCl₂ | 0.10 | 91 | 91 | 79 | 66 |
| Example 21 | Pb(NO₃)₂ | 0.10 | 93 | 93 | 90 | 89 |
| Example 22 | Sc(NO₃)₃ | 0.02 | 90 | 90 | 88 | 66 |
| Example 23 | La(NO₃)₃ | 0.02 | 91 | 91 | 87 | 70 |
| Comparison Example 24 | ZrOCl₂ | 0.05 | 90 | 90 | 87 | 49 |
| Example 25 | NH₄VO₃ | 0.05 | 87 | 87 | 80 | 38 |
| Example 26 | Cr(NO₃)₃ | 0.10 | 88 | 88 | 76 | 51 |
| Example 27 | SbCl₃ | 0.10 | 92 | 92 | 91 | 91 |
| Example 28 | Bi(NO₃)₃ | 0.10 | 89 | 89 | 88 | 86 |

## Claims

1. A process for producing an α-hydroxycarboxylic acid amide which comprises reacting an α-hydroxy nitrile compound with water in a liquid phase in the presence of a catalyst comprising manganese dioxide containing an element selected from the group consisting of Groups IIIA, VA (excluding nitrogen), IIIB, VB, VIB and VIII of the Periodic Table, carbon, silicon, germanium, lead and mixtures thereof, the manganese dioxide having been prepared by reacting permanganate with manganese sulphate under acidic conditions, the element contained in the manganese dioxide being present in an amount of from 0.005 to 0.5 atoms per atom of manganese and having been incorporated into the manganese dioxide by impregnation or kneading.

2. The process according to claim 1 in which the α-hydroxynitrile compound is acetone cyanohydrin.

3. The process according to claim 1 or claim 2 in which the catalyst is manganese dioxide containing an element of Group IIIA of the Periodic Table.

4. The process according to claim 1 or claim 2 in which the catalyst is manganese dioxide containing an element of Group VA of the Periodic Table (excluding nitrogen).

5. The process according to claim 1 or claim 2 in which the catalyst is manganese dioxide containing an element of Group IIIB of the Periodic Table.

6. The process according to claim 1 or claim 2 in which the catalyst is manganese dioxide containing an element of Group VIII of the Periodic Table.

7. The process according to claim 1 or claim 2 in which the catalyst is manganese dioxide containing lead.

8. The process according to claim 1 or claim 2 in which the catalyst is manganese dioxide containing antimony.

9. The process according to claim 1 or claim 2 in which the catalyst is manganese dioxide containing bismuth.

10. The process according to claim 1 or claim 2 in which the catalyst is manganese dioxide containing iron.

11. The process according to claim 1 or claim 2 in which the catalyst is manganese dioxide containing cobalt.

12. The process according to claim 1 or claim 2 in which the catalyst is manganese dioxide containing nickel.

## Patentansprüche

1. Verfahren zur Herstellung eines α-Hydroxycarbonsäureamids, umfassend die Umsetzung einer α-Hydroxynitril-Verbindung mit Wasser in einer flüssigen Phase in Gegenwart eines Mangandioxid-Katalysators, der ein aus der Gruppe ausgewähltes Element enthält, die aus den Gruppen IIIa, Va (mit Ausnahme von Stickstoff), IIIb, Vb, VIb und VIII des Periodensystems, aus Kohlenstoff, Silizium, Germanium, Blei und Gemischen davon besteht, wobei das Mangandioxid durch Reaktion von Permanganat mit Mangansulfat unter sauren Bedingungen hergestellt wird, wobei das im Mangandioxid enthaltene Element in einer Menge von 0,005 - 0,5 Atomen pro Mangan-Atom vorliegt und durch Tränken oder Kneten in das Mangandioxid eingearbeitet wurde.

2. Verfahren nach Anspruch 1, worin die α-Hydroxynitril-Verbindung Acetoncyanhydrin ist.

3. Verfahren nach Anspruch 1 oder 2, worin der Katalysator aus Mangandioxid besteht, das ein Element der Gruppe IIIa des Periodensystems enthält.

4. Verfahren nach Anspruch 1 oder 2, worin der Katalysator aus Mangandioxid besteht, das ein Element der Gruppe Va des Periodensystems enthält (mit Ausnahme von Stickstoff).

5. Verfahren nach Anspruch 1 oder 2, worin der Katalysator aus Mangandioxid besteht, das ein Element der Gruppe IIIb des Periodensystems enthält.

6. Verfahren nach Anspruch 1 oder 2, worin der Katalysator aus Mangandioxid besteht, das ein Element der Gruppe VIII des Periodensystems enthält.

7. Verfahren nach Anspruch 1 oder 2, worin der Katalysator aus bleihältigem Mangandioxid besteht.

8. Verfahren nach Anspruch 1 oder 2, worin der Katalysator aus antimonhältigem Mangandioxid besteht.

9. Verfahren nach Anspruch 1 oder 2, worin der Katalysator aus wismuthältigem Mangandioxid besteht.

10. Verfahren nach Anspruch 1 oder 2, worin der Katalysator aus eisenhältigem Mangandioxid besteht.

11. Verfahren nach Anspruch 1 oder 2, worin der Katalysator aus kobalthältigem Mangandioxid besteht.

12. Verfahren nach Anspruch 1 oder 2, worin der Katalysator aus nickelhältigem Mangandioxid besteht.

## Revendications

1. Procédé de production d'un amide d'acide α-hydroxycarboxylique qui comprend la mise en réaction d'un composé d'α-hydroxynitrile avec de l'eau dans une phase liquide en présence d'un catalyseur comprenant du dioxyde de manganèse contenant un élément sélectionné dans le groupe consistant en Groupes IIIA, VA (en excluant l'azote), IIIB, VB, VIB et VIII de la Table Périodique, le carbone, le silicium, le germanium, le plomb et des mélanges de ceux-ci, le dioxyde de manganèse ayant été préparé en faisant réagir du permanganate avec du sulfate de manganèse en conditions acides, l'élément contenu dans le dioxyde de manganèse étant présent en une quantité de 0,005 à 0,5 atome par atome de manganèse et ayant été incorporé dans le dioxyde de manganèse par imprégnation ou malaxage.

2. Procédé selon la revendication 1 dans lequel le composé d'α-hydroxynitrile est de la cyanohydrine d'acétone.

3. Procédé selon la revendication 1 ou la revendication 2 dans lequel le catalyseur est du dioxyde de manganèse contenant un élément du Groupe IIIA de la Table Périodique.

4. Procédé selon la revendication 1 ou la revendication 2 dans lequel le catalyseur est du dioxyde de manganèse contenant un élément du Groupe VA de la Table Périodique (excluant l'azote).

5. Procédé selon la revendication 1 ou la revendication 2 dans lequel le catalyseur est du dioxyde de manganèse contenant un élément du Groupe IIIB de la Table Périodique.

6. Procédé selon la revendication 1 ou la revendication 2 dans lequel le catalyseur est du dioxyde de manganèse contenant un élément du Groupe VIII de la Table Périodique.

7. Procédé selon la revendication 1 ou la revendication 2 dans lequel le catalyseur est du dioxyde de manganèse contenant du plomb.

8. Procédé selon la revendication 1 ou la revendication 2 dans lequel le catalyseur est du dioxyde de manganèse contenant de l'antimoine.

9. Procédé selon la revendication 1 ou la revendication 2 dans lequel le catalyseur est du dioxyde de manganèse contenant du bismuth.

10. Procédé selon la revendication 1 ou la revendication 2 dans lequel le catalyseur est du dioxyde de manganèse contenant du fer.

11. Procédé selon la revendication 1 ou la revendication 2 dans lequel le catalyseur est du dioxyde de manganèse contenant du cobalt.

12. Procédé selon la revendication 1 ou la revendication 2 dans lequel le catalyseur est du dioxyde de manganèse contenant du nickel.
